# EUROPÄISCHE PATENTANMELDUNG

(11) **EP 1 312 309 A1**
(43) Veröffentlichungstag der Anmeldung: **21.05.2003**
(21) Anmeldenummer: 01126789.5
(22) Anmeldetag: 09.11.2001
(51) Int. Cl.: A61B 8/08, A61B 6/03

(54) **Verfahren zur Bestimmung der Lage eines Objekts anhand von ortsaufgelösten Bilddaten**

(71) Anmelder: ZN Vision Technologies AG, 44801 Bochum (DE)
(72) Erfinder: Brendel, Bernhard, 44795 Bochum (DE); Winter, Susanne, 44892 Bochum (DE); Rick, Andreas, 58239 Schwerte (DE)
(74) Vertreter: Grünecker, Kinkeldey, Stockmair & Schwanhäusser Anwaltssozietät

(57) **Zusammenfassung**

Die Erfindung betrifft ein Verfahren zur Bestimmung der Lage eines Objektes oder eines Objektbereichs unter Verwendung eines mit einem ersten Bilderzeugungsverfahren erstellten ortsaufgelösten Referenzbilddatensatzes für ein Referenzbild und eines zweiten Bilderzeugungsverfahrens, insbesondere zur Bestimmung der Lage von Gewebeteilen und/oder Organen und/oder der Organstruktur bei einer navigierten Operation, mit den Schritten Identifizieren solcher im Referenzbild abgebildeter Bildbereiche, die bei dem zweiten Bilderzeugungsverfahren ein charakteristisches Abbildungsverhalten aufweisen, Verwenden des zweiten Verfahrens zur Bilderzeugung zum Erzeugen eines weiteren ortsaufgelösten Bilddatensatzes für ein weiteres Bild des Objektes, Ermitteln der Transformation zwischen dem weiteren Bilddatensatz und dem Referenzbilddatensatz, welche die identifizierten Bildbereiche in bestmögliche Übereinstimmung bringt, und Bestimmen der Lage des Objekts oder des Objektbereichs in der dem weiteren Bild entsprechenden Situation.

## Beschreibung

Die Erfindung betrifft ein Verfahren zur Referenzierung von ortsaufgelösten Bilddaten. Insbesondere kann die Erfindung zur Referenzierung von dreidimensionalen Bilddaten von Körperteilen, Organen, Knochen oder anderer Gewebestrukturen mit Ultraschallbildern verwendet werden.

### Stand der Technik

In der Chirurgie werden Computertomographie- oder Magnetresonanztomographieaufnahmen häufig präoperativ aufgenommen und zur Planung von Eingriffen herangezogen. Dabei kommt je nach Art und Ort des geplanten Eingriffs eines der obengenannten bildgebenden Verfahren zur Anwendung, die entsprechend der räumlichen Auflösung und der Kontrastauflösung eine bestmögliche Darstellung der Gewebebereiche, die für den Eingriff relevant sind, ermöglicht.

So werden zur Operation an Knochen häufig Computertomographieaufnahmen angefertigt, da hier sowohl die räumliche Auflösung sehr hoch ist, als auch die Knochen einen hohen Kontrast zu anderem Gewebe aufweisen.

Bei Operationen an weichen Gewebeteilen wird häufig eine Magnetresonanztomographie angewendet, da hierbei der Kontrast in diesen Gewebeteilen besser ist.

Die beiden obengenannten Bilderzeugungsverfahren eignen sich nur bedingt zur intraoperativen Bildgebung, da sowohl der Computertomograph, als auch der Magnettomograph den Zugang zum Patienten deutlich erschweren.

Zusätzlich belastet der Computertomograph das behandelnde Personal durch die ionisierende Röntgenstrahlung, wenn sie während der Bildaufnahme in der Nähe des Patienten bleiben müssen.

Selbst wenn bei Magnetresonanztomographiesystemen mit offenen Magneten ein Zugang zum Patienten freigegeben ist, wird die Operation durch das Magnetfeld erschwert, so dass nur ausgewählte Materialien bei der Operation verwendet werden können. Als zusätzlicher Nachteil erweisen sich die hohen Kosten sowohl der Tomographen, als auch der notwendigen speziellen Ausrüstung des Operationssaals.

Aus diesem Grund werden beide bildgebenden Verfahren in der Chirurgie nur sehr selten intraoperativ verwendet.

In der Druckschrift DE 19951502 A1 ist eine gemeinsame Darstellung intraoperativ aufgenommener Bilddaten mit Operationsinstrumenten skizziert. Die Nachteile dabei sind allerdings die relativ zu den präoperativ aufgenommenen Referenzbilddaten niedrige Bildqualität der intraoperativen Bilder, sowie die hohe Strahlenbelastung des behandelnden Personals bei Verwendung von Röntgenstrahlung.

Um die Vorteile von bildgebenden Verfahren auch während der Operation nutzen zu können, werden in der Praxis Navigationssysteme verwendet, die es ermöglichen, die präoperativ aufgenommenen Referenzbilddaten mit der Lage des Patienten sowie der Operationsinstrumente während eines Eingriffs in Beziehung zu bringen.

Das Navigationssystem ermöglicht durch geeignete Sensortechnik, z.B. durch optische, magnetische oder induktive Sensoren, die Position von Instrumenten zu verfolgen und in einem gemeinsamen Koordinatensystem zu bestimmen.

Um diese Positionsangaben in Verbindung mit den präoperativ aufgenommenen Referenzbilddaten nutzen zu können, müssen die Koordinatensysteme des Navigationssystems und des Bilddatensatzes referenziert werden, das heißt, die Umrechnungsvorschrift (Koordinatentransformation) von einem Punkt im Koordinatensystem des Navigationssystems in das Koordinatensystem des Referenzbilddatensatzes muss bestimmt werden. Ferner muss berücksichtigt werden, dass sich ein Patient und biologisches Gewebe im Allgemeinen nicht wie ein starrer Körper verhalten, sondern ihre Form und/oder ihre Ausdehnung in einem gewissen Rahmen ändern können: Bewegungen eines Patienten, aber auch ganz normale Funktionsprozesse wie Atmung, Herzschlag, Verdauung, etc. können sowohl die relative Lage von Organen zueinander verändern, als auch die Größe und Form jedes Organs für sich verändern.

Die Rechenoperation, die sowohl den Koordinatenwechsel, als auch die zuletzt genannten Positions- und Formänderungen berücksichtigt, wird in dieser Anmeldung als Transformation bezeichnet.

Es gibt verschiedene Verfahren zur Referenzierung der präoperativen Referenzbilddaten mit dem Koordinatensystem des Navigationssystems, die sich zum einen in der Art des Sensors unterscheiden, mit deren Hilfe die Position des Organs oder Gewebeteils während der Operation gemessen wird, und zum anderen in der Methode der Berechnung der Transformation aus diesen Sensordaten.

Besonders günstig für die Verwendung während der Operation haben sich Sensoren erwiesen, die klein und handlich sind und daher auch während der gesamten Operation leicht eine wiederholte Registrierung ermöglichen. Dadurch kann vor einer kritischen Aktion während der Operation eine Verifizierung der Lage des Patienten relativ zum Navigationssystem durchgeführt werden, um eine Fehlpositionierung durch Patientenbewegung zu vermeiden.

In der Druckschrift US 6,006,126 werden hierzu optische Sensoren verwendet, bei denen entweder die Position der Patientenoberfläche oder eines am Patienten angebrachten Zeigers durch mehrere Kameras bestimmt wird.

Eine andere Möglichkeit ist es, Tastköpfe zu verwenden, mit denen die Oberfläche von zugänglichen Körperstrukturen abgetastet oder abgefahren werden können.

Sowohl Tastköpfe, als auch die optischen Sensoren haben aber den Nachteil, dass sie nicht an im Inneren des Patienten liegenden Stellen angewendet werden können, ohne einen Zugang durch darüber liegende Gewebestrukturen zu schaffen.

Um dieses Problem zu vermeiden, beschreiben die Druckschriften US 6,106,464; "Clinical validation of percutaneous computer assisted surgery using ultrasound", J. Tonetti, L.Carrat, S. Blendea, J. Troccaz, P. Merloz, S.Lavallee, Ist. Annual Meeting of the International Society for Computer Assisted Orthopaedic Surgery, Feb. 7-10, 2001, Davos, Switzerland, S. 37; "On B-Mode Ultrasound based registration for computer assisted orthobaedic surgery", J. Kowal, C. A. Amstutz, L.P. Nolte, ibid., S. 35, "Ultrasound based registration of the pelvic bone surface for surgical navigation", D.V. Amin, T. Kanade, A.M. Digioia, B. Jaramaz, C. Nikou, R.S. Labarca, ibid., S.35, die Verwendung eines Ultraschallkopfs als Sensor.

Dieser Ultraschallkopf ermöglicht es in einigen Anwendungsfällen, die Knochenoberfläche auch durch darüber liegende Gewebeschichten zu erkennen und daraus die Position des Knochens zu bestimmen.

Diese Verfahren leiden aber unter dem schlechten Signal/Rausch-Abstand der Ultraschallsensorik. Die Methode kann daher nur auf Knochen angewendet werden, da für andere Körperteile der Kontrast an der Organgrenze nicht für eine brauchbare Segmentierung ausreicht. Wegen des sogenannten "Speckle" kann aber auch eine Knochenoberfläche nicht in jedem Fall sicher im Ultraschallbild erkannt werden, so dass die Methode nur bei sehr dicht unter der Haut liegenden Knochen angewendet werden kann.

Es ist eine Aufgabe der vorliegenden Erfindung, ein Verfahren bereitzustellen, welches die Nachteile der im Stand der Technik beschriebenen Verfahren vermeidet.

Insbesondere ist es eine Aufgabe der Erfindung, ein Verfahren bereitzustellen, mit dem es möglich wird, die aktuelle räumliche Lage von Objekten oder Objektbereichen, insbesondere von Gewebeteilen oder Organen von Lebewesen und/oder deren Organstruktur, zuverlässig mit Hilfe eines Messverfahrens zu ermitteln, welches zwar problemlos in jeder aktuellen Situation einsetzbar ist, aber für sich allein zur Darstellung der räumlichen Lage nicht geeignet ist.

Diese Aufgabe wird durch die Verfahren mit den in den Anspruch 1 angegebenen Schritten gelöst. Vorteilhafte Weiterführungen werden in den Unteransprüchen beschrieben.

Demzufolge stellt die Erfindung ein Verfahren zur Bestimmung der Lage eines Objektes oder eines Objektbereichs unter Verwendung eines mit einem ersten Bilderzeugungsverfahren erstellten ortsaufgelösten Referenzbilddatensatzes für ein Referenzbild und eines zweiten Bilderzeugungsverfahrens bereit, insbesondere zur Bestimmung der Lage von Gewebeteilen und/oder Organen und/oder der Organstruktur bei einer navigierten Operation, das die folgenden Schritte aufweist:

Identifizieren solcher im Referenzbild abgebildeter Bildbereiche, die bei dem zweiten Bilderzeugungsverfahren ein charakteristisches Abbildungsverhalten aufweisen, Verwenden des zweiten Verfahrens zur Bilderzeugung zum Erzeugen eines weiteren ortsaufgelösten Bilddatensatzes für ein weiteres Bild des Objektes, Ermitteln der Transformation zwischen dem weiteren Bilddatensatz und dem Referenzbilddatensatz, welche die identifizierten Bildbereiche in bestmögliche Übereinstimmung bringt, und Bestimmen der Lage des Objekts oder des Objektbereichs in der dem weiteren Bild entsprechenden Situation.

Es sei darauf hingewiesen, dass in dieser Anmeldung anstelle der unhandlichen Begriffe "Referenzbilddatensatz" und "Bilddatensatz" häufig die prägnanteren Ausdrücke "Referenzbild" und "Bild" verwendet werden, ohne dass dies automatisch eine bildliche Darstellung implizieren soll. Wenn bei der Verwendung der Begriffe "Referenzbild"/"Bild" tatsächlich eine visuelle Darstellung des entsprechenden Datensatzes, d.h. eine Bildwiedergabe, gemeint ist, so ist dies entweder explizit angegeben oder geht unzweideutig aus dem Kontext hervor.

Aus der ermittelten Transformation zwischen dem weiteren Bild und dem Referenzbild, welche die identifizierten Bildbereiche in bestmögliche Übereinstimmung bringt, und aus der entsprechenden Umkehrtransformation kann die dem weiteren Bild entsprechende aktuelle Situation wahlweise durch einen Bilddatensatz im Koordinatensystem des ersten oder zweiten Bilderzeugungsverfahrens dargestellt werden.

Mit "räumlicher Lage" ist die Position und/oder Orientierung des Objekts oder des Objektbereichs in einem bestimmten Koordinatensystem gemeint. Dieses bestimmte Koordinatensystem kann sich von den entsprechenden Koordinatensystemen der ersten und zweiten Bilderzeugungsverfahren unterscheiden. Es ist vorzugsweise so gewählt, dass die Visualisierung der Objekte/Objektbereiche in diesem bestimmten Koordinatensystem die im weiteren Bild aufgenommene Situation aus dem Blickwinkel einer Person, beispielsweise eines behandelnden Arztes wiedergibt. Alternativ kann es auch ein Koordinatensystem mit einem Operationsgerät als Bezugspunkt sein.

Die räumliche Lage in diesem bestimmten Koordinatensystem geht aus der räumlichen Lage in den ersten beiden Koordinatensystemen durch eine einfache Koordinatentransformation hervor.

Der Vorteil des erfindungsgemäßen Verfahrens ist, dass es die jeweiligen Vorteile des ersten und des zweiten Bilderzeugungsverfahrens nutzen und gleichzeitig die jeweiligen Nachteile vermeiden kann.

Das erste Bilderzeugungsverfahren umfasst solche Verfahren wie Tomographie, Röntgenverfahren, etc., die Bilder mit hoher Detaillierung und in mehreren Dimensionen gute Ortsauflösung liefern, aber aus unterschiedlichsten Gründen in der Regel nicht über einen längeren Zeitraum, z.B. während einer Operation verwendet werden. Eine exemplarische Auswahl solcher Gründe ist nachfolgend angegeben: Zeitaufwändigkeit, Gesundheitsrisiken für Bedienpersonal (und gegebenenfalls untersuchte Personen), großer Platzbedarf, Unzugänglichkeit, etc.

Im Gegensatz dazu ist das zweite Bilderzeugungsverfahren weder zeit- noch platzaufwändig, birgt kaum Gesundheitsrisiken für Bedienpersonal (und gegebenenfalls untersuchte Personen), kann also *in situ* verwendet werden, allerdings ist der durch es gelieferte Informationsgehalt vergleichsweise gering. Ein typisches Beispiel hierfür sind Ultraschallverfahren.

Diese beiden Verfahren werden durch die Erfindung so kombiniert, dass anfänglich ein Referenzbild hoher Auflösung und mit hohem Detailreichtum durch das erste Verfahren erzeugt wird, nachfolgend ein weiteres Bild mit dem zweiten Verfahren erzeugt wird, solche Bereiche im Referenzbild identifiziert werden, die auch im weiteren Bild deutlich abgebildet sein müssten, und eine Transformation ermittelt wird, die eine möglichst gute Abbildung der im Referenzbild identifizierten Bildbereiche auf das weitere Bild erlaubt.

Diese Transformation umfasst sowohl einfache Koordinatentransformationen und Skalierungsoperationen, die notwendig sind, wenn die beiden Bilderzeugungsverfahren Objekte aus verschiedenen Richtungen und Abständen "sehen", als auch Rechenoperationen, welche Form- und Lageänderungen der Objekte berücksichtigen.

Durch Anwenden dieser Transformation auf den Referenzbilddatensatz kann demzufolge ein aktualisierter Bilddatensatz erzeugt werden, der dem Aufnahmezeitpunkt des weiteren Bildes entspricht, aber eine Ortauflösung und/oder einen Detailreichtum besitzt aufweist, die das weitere Bild nicht oder nur ungenügend aufweist.

In diesem aktualisierten Bilddatensatz kann somit die Lage des Objekts oder eines interessierenden Objektbereichs mit höherer Präzision als im weiteren Bild, unter Umständen sogar überhaupt erst festgestellt werden. Dasselbe kann für jedes nachfolgende Bild analog durchgeführt werden.

Vorzugsweise weist das Referenzbild eine dreidimensionale Ortsauflösung auf und jedes weitere Bild wenigstens eine zweidimensionale Ortsauflösung. Dadurch kann die Lage eines Objekts/Objektbereichs selbst dann vollständig im dreidimensionalen Raum ermittelt werden, wenn sie frei im Raum bewegbar sind.

Eine niedriger dimensionale (beispielsweise eine zweidimensionale) Ortsauflösung des Referenzbildes ist ausreichend, wenn sich Bewegungen und andere Veränderungen im wesentlichen nur auf bestimmte Raumrichtungen oder Ebenen im Raum beschränken.

Als Referenzbild wird besonders bevorzugt ein durch Computertomographie, Röntgentomograhie oder Magnetresonanztomographie erzeugtes Bild verwendet, während als zweites, "in situ" Bilderzeugungsverfahren besonders vorteilhaft ein Ultraschallverfahren eingesetzt werden kann.

Im speziellen Fall der Verwendung des erfindungsgemäßen Verfahrens für eine navigierte Operation werden anhand der Bilddaten des präoperativ aufgenommenen Referenzbilddatensatzes Bereiche bestimmt, von denen bekannt ist, dass sie beim zweiten Verfahren, beispielsweise im Ultraschallbild, ein charakteristisches Signal ergeben. Die Bereiche können lokale Punkte oder ausgedehntere Regionen sein. Das charakteristische Signal wird für diese Bereiche abgeschätzt und die Position der bestmöglichen Übereinstimmung zwischen dem geschätzten Signal und dem Ultraschallbildsignal wird ermittelt.

Die Bestimmung der optimalen Position wird mit Hilfe eines Optimierungsverfahrens, beispielsweise durch Gradientenabstieg, stochastische Methoden oder genetische Optimierungsverfahren durchgeführt.

Durch die Reduzierung des Vergleichs auf die Punkte oder Regionen, von denen das charakteristische Signal im Ultraschall bekannt ist, kann die Rechenzeit bei Implementierung des Verfahrens auf einem Rechner durch die Reduktion der Anzahl nötiger Vergleiche gegenüber Verfahren, die zur Registrierung sowohl den gesamten weiteren Bilddatensatz, als auch den gesamten präoperativen Referenzbilddatensatz verwenden, deutlich reduziert werden, gegebenenfalls so stark, dass eine Registrierung in Echtzeit ermöglicht wird.

Für den Fall der Ultraschallbildgebung wird der weitere Vorteil erreicht, dass Rauschen (Speckle) wenig Einfluss auf das Optimierungsergebnis hat.

Da die automatische Auswahl von Bildbereichen (Punkte oder Regionen) aus dem präoperativ aufgenommenen Referenzbilddatensatz, von denen das charakteristische Signal im Ultraschall bekannt ist, es im Vergleich mit Verfahren, die manuell wenige ausgewählte Registrierungspunkte verwenden, ermöglicht, eine Vielzahl von Punkten zur Referenzierung mit dem Ultraschallbild heranzuziehen, ist das erfindungsgemäße Verfahren deutlich robuster gegenüber Rauschen und anderen Störungen.

In verschiedenen, je nach Situation und Einsatzgebiet bevorzugten Weiterbildungen erzeugt das Ultraschallverfahren Messsignale mit ein-, zwei- oder dreidimensionaler Ortsauflösung.

Im Fall von eindimensional ortsaufgelösten Messsignalen können zwei- oder dreidimensionale Bilder durch Überlagerung einer Mehrzahl der eindimensional ortsaufgelösten Messsignale erzeugt werden. Bei Oberflächenuntersuchungen können die Messsignale mit zweidimensionaler Ortsauflösung direkt zur Bilderzeugung verwendet werden. Andernfalls können auch hier mehrere zweidimensionale Schnitte zum Aufbau eines dreidimensionalen Bildes herangezogen werden.

In einer besonders vorteilhaften Weiterbildung umfasst das Erzeugen des wenigstens einen weiteren ortsaufgelösten Bildes des Objektes ein Bearbeiten der mit dem zweiten Bilderzeugungsverfahren erzeugten Messsignale, so dass die Bereiche mit charakteristischem Abbildungsverhalten hervorgehoben werden. Bevorzugte Bearbeitungsverfahren sind das Durchführen von punktweisen Grauwerttransformationen, beispielsweise auf Grundlage einer quadratischen Kennlinie.

Ebenfalls zur Eliminierung von unsicherer (weil zu verrauschter) oder überflüssiger (weil für den jeweiligen Zweck nicht gebrauchter) Information dient ein weiteres Bearbeitungsverfahren, bei dem die Messsignale einer räumlichen Filterung unterzogen werden, wodurch derartige Informationen aus der Bildgebung aussortiert werden.

In einer besonders vorteilhaften Weiterbildung des erfindungsgemäßen Verfahrens erfolgt die Ermittlung der Transformation durch Optimierung eines Gütekriteriums, das auf dem identifizierten Bildbereich berechnet wird und dieser Bereich so identifiziert wurde, dass er die Oberfläche im dreidimensionalen Referenzbild darstellt, wobei der die identifizierten Oberflächen darstellende Datensatz aus dem dreidimensionalen Referenzbilddatensatz berechnet wird. Ein solches Vorgehen ist sinnvoll, wenn das zweite Bilderzeugungsverfahren ein für diese Oberflächen sensitives Verfahren ist, und insbesondere dann, wenn das zweite Bilderzeugungsverfahren wenig oder keine Information über Bereiche unterhalb/innerhalb dieser Oberflächen liefert.

Vorzugsweise wird der die Oberflächen darstellende Datensatz durch ein Segmentierungsverfahren aus dem dreidimensionalen Referenzbilddatensatz berechnet. Im einfachsten Fall kann die Segmentierung durch Schwellwertbildung realisiert werden. So wird beim besonders repräsentativen Beispiel eines Knochens der Schwellwert so gewählt, dass die durch den Datensatz dargestellte Oberfläche die Knochenoberfläche wiedergibt.

Ein solches Vorgehen ist wiederum vor allem bei Ultraschalluntersuchungen empfehlenswert, wobei es zudem sinnvoll ist, das Identifizieren von Bildbereichen in dem Referenzbild nur auf solchen Oberflächenbereichen durchzuführen, die einem zur Durchführung des zweiten Verfahrens verwendeten Ultraschallkopf zugewandt sind, da ihm abgewandte Oberflächenbereiche von Objekten im Objektschatten liegen und die Ultraschalluntersuchung keinerlei brauchbare Information liefert.

Besonders zuverlässig können Transformationen zwischen den Ultraschallbilddaten und den Referenzbilddaten unter Verwendung von Gewichtsfaktoren ermittelt werden, wenn diese aus den Normalenvektoren an Punkten der Oberfläche und den entsprechenden Vektoren der Ausbreitungsrichtung des Ultraschalls berechnet werden. Insbesondere eignen sich hierzu Skalarprodukte zwischen diesen Vektoren. Je größer hierbei der Wert des Skalarprodukts ist, d.h. je frontaler der Oberflächenbereich dem Ultraschallkopf zugewandt ist, desto aufschlussreicher sind die durch den Ultraschall gewonnen Daten. Deshalb sind die Gewichtsfaktoren eine monoton zunehmende - im einfachsten Fall: lineare - Funktion des Skalarproduktes.

Bei einem Ultraschallverfahren als zweitem bildgebenden Verfahren werden für das Identifizieren von Bildbereichen im Referenzbild vorzugsweise all jene Objektbereiche - im Speziellen: Organe - berücksichtigt, die im Ultraschallbild eine Abschattung erzeugen. Dabei ist es zweckmäßig, die Ermittlung der Transformation zwischen den Ultraschallbilddaten und den Referenzbilddaten so durchzuführen, dass die Summe der Grauwerte im Ultraschallbild innerhalb der Bildbereiche minimiert wird.

Die oben beschriebenen Verfahren sind besonders vorteilhaft in einem System zur navigierten Operation einsetzbar, dass wenigstens einen Ultraschallsensor verwendet, um aus einem präoperativ aufgenommenen Referenzbild und wenigstens einem aktuellen Ultraschallbild die aktuelle Lage von Gewebeteilen und/oder Organen und/oder der Organstruktur zu ermitteln.

Sie sind insbesondere dafür verwendbar, um einen aktuellen Bilddatensatz mit einem Informationsgehalt, der vergleichbar mit dem des Referenzbilddatensatzes ist, auf Grundlage des ("veralteten") Referenzbildes und der ermittelten aktuellen Transformation zu berechnen, und darüber hinaus das dem aktuellen Bilddatensatz entsprechende aktuelle Bild visuell darzustellen.

Besonders vorteilhaft für Chirurgie und andere operative Methoden ist eine Weiterbildung, bei der im visuell dargestellten aktuellen Bild chirurgische und andere medizinische Instrumente gemeinsam mit den Gewebeteilen und/oder den Organen und/oder der Organstruktur abgebildet werden. In diesem Fall muss ein Arzt mit seiner Aufmerksamkeit nicht zwischen Bildschirm und Patienten hin- und herschwenken, sondern kann sich im wesentlichen auf den Bildschirm konzentrieren. Dadurch ist eine "Operation am Bildschirm" möglich.

Für den Fall, dass ein Patient nicht voll narkotisiert ist und Bewegungen durchführen kann, oder dass er bewegt, z.B. gedreht, werden muss, müssen notwendigerweise weitere ortsaufgelöste Bilder während der Bewegung des Patienten aufgenommen werden und die sukzessiv aufgenommenen aktuellen Bilddatensätze zur Darstellung der Bewegungsphase des Patienten verwendet werden. Dabei sollte die Visualisierung nur wenig zeitversetzt sein und nach Möglichkeit nur Sekundenbruchteile hinter der Realität hinterherhinken, so dass eine Darstellung in Echtzeit oder nahezu in Echtzeit erfolgen kann.

### Ausführliche Beschreibung der Erfindung

Die beigefügten schematischen Zeichnungen legen Ausführungsbeispiele der Erfindung dar. Dabei zeigen:
Fig 1.: eine Übersicht eines Systems zur Verwendung des erfindungsgemäßen Verfahrens mit einem Patienten,
Fig 2: ein Ablaufschema der Registrierung,
Fig 3: die Bestimmung des Skalarprodukts zwischen Oberflächennormalenvektor und dem Vektor der Ultraschallausbreitungsrichtung,

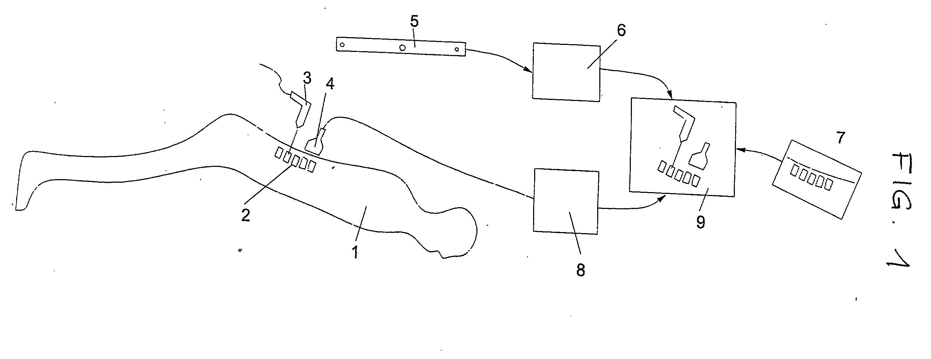

In der Figur 1 ist eine Übersicht der Anwendung des erfindungsgemäßen Verfahrens zu navigierte Operation an der Wirbelsäule dargestellt. Der Patient 1 mit der Wirbelsäule 2 die bei der Operation durch Bohrung von Löchern zur Befestigung von Pedikelschrauben mit Hilfe des Operationsinstruments 3 verändert werden soll, liegt auf einem nicht dargestellten Operationstisch.

Der für den Eingriff wichtige Bereich 2 des Patienten 1 wird mit Hilfe des Ultraschallkopfes 4 und des Ultraschallgerätes 8 abgetastet, wobei gleichzeitig die Position des Ultraschallkopfes 4 durch das hier exemplarisch dargestellte optische Sensorsystem 5 aufgenommen wird und über das Positionsbestimmungssystem 6 dem Navigationssystem 9 zugeführt wird. Die weiteren Bilddaten werden vom Ultraschallsystem 8 ebenfalls an das Navigationssystem 9 übergeben.

Das Navigationssystem 9 bekommt außerdem den präoperativ aufgenommenen dreidimensionalen Referenzbilddatensatz 7 zugeführt. Das Navigationssystem 9 benutzt das erfindungsgemäße Verfahren zur Bestimmung der Transformation von dem dreidimensionalen Datensatz 7 und dem Koordinatensystem des Navigationssystems 6, um das Operationsinstrument 3, dessen Position ebenfalls mit dem Sensorsystem 5 gemessen wird, zusammen mit den Bilddaten 7 des Patienten 1 und insbesondere den für den Eingriff interessierenden Bereich 2 gemeinsam darzustellen, so dass der Chirurg die nächsten Schritte seines Eingriffs genau vorausplanen kann und die Führung seines Operationsinstruments 3 unter Berücksichtigung einer eventuellen Bewegung des Patienten 1 optimal anpassen kann.

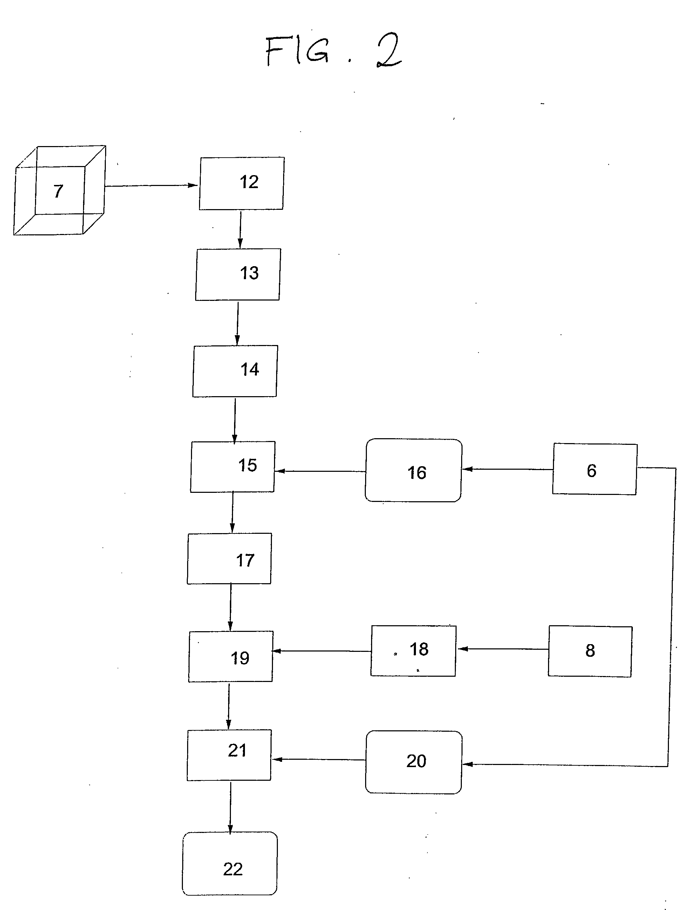

In Figur 2 ist beispielhaft ein Ausführung des erfindungsgemäßen Verfahrens für die in Figur 1 beschriebene Anwendung in den Teilschritten dargestellt.

In dem präoperativ aufgenommene Referenzbilddatensatz 7 wird durch eine Schwellwertsegmentierung 12 die Wirbelsäule von den umliegenden Gewebestrukturen getrennt. Ein zweiter Segmentierungsschritt 13 trennt die Wirbelsäule in die Regionen der einzelne Wirbel auf.

Mit Hilfe einer Oberflächenrekonstruktion 14 wird die Oberfläche der Wirbelkörper berechnet.

In der folgenden Winkelbestimmung 15 wird mit Hilfe der Positionsdaten 16 des Positionsbestimmungssystems 6 eine erste Abschätzung der Einstrahlungsrichtung des Ultraschallsignals in das CT Volumen und daraus der Winkel zwischen den Oberflächenelemente aus der Oberflächenrekonstruktion 14 und der Ultraschallausbreitungsrichtung berechnet. Dies kann z.B. durch eine grobe Vorpositionierung des präoperativen Datensatzes relativ zum Koordinatensystem des Positionsbestimmungssystems 6 durch den Benutzer erfolgen.

Durch eine Kalibrierung des Ultraschallkopfes und des an ihm eventuell angebrachten Teils des Sensorsystems 5, die vor Beginn der Operation oder bei der Herstellung des Systems erfolgt, ist die Ausbreitungsrichtung des Ultraschalls in den Koordinaten des Positionsbestimmungssystems 6 bekannt. Aus diesen Daten kann durch einfache geometrische Berechnungen der Winkel zwischen dem Ultraschallsignal und den Oberflächenelemente aus der Oberflächenrekonstruktion 14 bestimmt werden.

Der Oberflächenreduzierungsschritt 17 selektiert aus diesen Daten nur diejenigen Oberflächenelemente, die in einem vorgegebenen Winkelbereich liegen und vom Ultraschallkopf aus sichtbar sind, so dass nur Oberflächenelemente in die folgenden Schritte eingehen, die den Ultraschall in Richtung des Ultraschallkopfes zurückwerfen.

In einer anderen Ausprägung des erfindungsgemäßen Verfahrens werden die Schritte 14, 15 und 17 zusammengefasst, indem alle Oberflächenelemente mit einem Normalenvektor in einem fester Winkelbereich zur möglichen Einstrahlungsrichtung des Ultraschalls berechnet werden.

Als ein Beispiel, ohne Einschränkung der Allgemeinheit, können bei Aufnahmen von der Wirbelsäule alle dorsal sichtbaren Oberflächenelemente der Wirbelkörper mit einem Winkel zwischen Normalenvektor und einer Achse senkrecht auf dem Rücken des Patienten von kleiner als 45 Grad verwendet werden.

Ein Beispiel für die Bearbeitung des Ultraschallsignals kann wie folgt aussehen: Die Bearbeitung des Ultraschallsignals 18 filtert das vom Ultraschallgerät 8 kommende Bild, so dass die Speckles reduziert werden. Es wird außerdem eine Kompensation des mit der Eindringtiefe abnehmenden Signalpegels der Ultraschalldaten vorgenommen.

Als Bearbeitungsfilter können beispielsweise räumliche Tiefpassfilter verwendet werden.

Eine weitere Möglichkeit besteht in der Verwendung von Kombinationsverfahren (engl. Compounding), bei dem mehrere Ultraschallbilder vom gleichen Gewebeteil mit unterschiedlicher Einstrahlungsrichtung in das Gewebe aufgenommen werden, und diese Bilder anschließend kombiniert werden.

Dabei hat sich die Kombination durch den Maximumoperator als besonders vorteilhaft erwiesen, weil dadurch die Bildschärfe nicht beeinträchtigt wird und das hohe Reflektionssignal an Oberflächenstrukturen erhalten bleibt.

Ein andere Möglichkeit der Bearbeitung besteht in der Grauwerttransformation der Grauwerte des Ultraschallsignals durch eine Kennlinie. Hierbei hat sich eine quadratische Kennlinie als günstig erwiesen, da diese die hohen Reflektionssignale an Oberflächenstrukturen verstärken.

Die Optimierungsfunktion 19 bestimmt die beste Transformation zwischen den Koordinatensystemen des Ultraschallbildes und des dreidimensionalen Datensatzes 7, indem die Oberflächenelemente eines Wirbelkörpers aus 17 rotiert und verschoben werden und die Summe über alle Punkte dieser Oberflächen im Ultraschalldatensatz gebildet wird und als Optimierungskriterium für ein Optimierungsverfahren eingesetzt wird, das diese Rotation und Verschiebung für jede Oberfläche optimiert.

Eine andere Ausführung verwendet die Summe der Grauwerte des weiteren Bilddatensatzes gewichtet mit den Skalarprodukten zwischen den Normalenvektoren der Oberflächenelemente und der Strahlungsrichtung des Ultraschalls über alle Punkte der Oberfläche, die in Strahlungsrichtung vom Ultraschallsensor sichtbar sind.

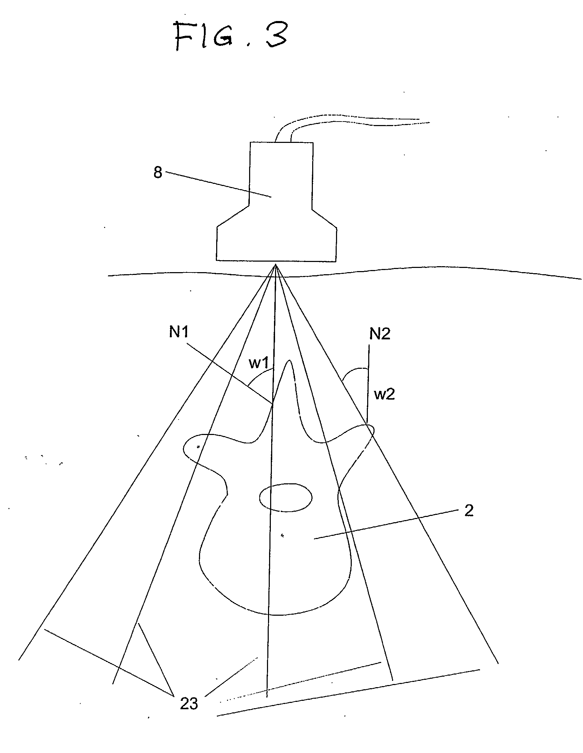

Figur 3 zeigt die Oberfläche des Gewebeteils 2 und einige exemplarische Ultraschallstrahlen 23 die vom Ultraschallwandler 8 ausgehen, sowie die Normalenvektoren N1 und N2 auf zwei Oberflächenelemente sowie die Winkel w1 und w2 zwischen den Normalenvektoren und der Ultraschallausbreitungsrichtung.

Im Falle von weichen Organen oder Gewebeteilen kann bei der Optimierung auch eine Deformation bestimmt werden.

Als Optimierungsverfahren können Gradientenverfahren angewendet werden, besonders wenn die ursprüngliche (initiale) Positionierung des dreidimensionalen Referenzbilddatensatzes durch den Benutzer eine gute erste Approximation der zu bestimmenden Transformation darstellt. Falls keine gute initiale Positionsbestimmung vorhanden ist, können stochastische Verfahren oder sogenannte genetische Verfahren erfolgreich angewendet werden, bei denen das Problem des Verharrens der Optimierungsschritte in lokalen Maxima der Optimierungsfunktion vermieden wird.

Durch die erfindungsgemäß günstige Auswahl der Punkte die zum Vergleich der Datensätze herangezogen werden, konvergiert der Optimierungsschritt im allgemeinen nach wenigen Iterationsschritten.

Das Darstellungssystem 21 verwendet die in 19 bestimmte Transformation, den dreidimensionalen Datensatz 7 und die Positionsdaten 20 des Operationsinstrumentes 3, um ein gemeinsames Bild 22 zu zeigen.

In einer anderen Anwendung des erfindungsgemäßen Verfahrens bei dem es um die Analyse der Bewegung des Patienten geht, werden eine Reihe von Ultraschallaufnahmen vom Patienten gemacht, während dieser sich bewegt.

Als Beispiel sei hier wieder die Wirbelsäule als der für den Arzt interessante Bereich angenommen.

Durch die mehrfache Berechnung der Koordinatentransformation entsprechend der in Figur 2 dargestellten Teilschritte kann für jede Bewegungsphase des Patienten die Position eines jeden Wirbels im Sichtbereich des Ultraschallkopfes bestimmt werden.

Durch Anwendung der Koordinatentransformation auf den vorher aufgenommenen dreidimensionalen Datensatz entstehen eine Reihe von verformten Datensätzen bei denen die Wirbelpositionen mit den Positionen der echten Wirbel des Patienten während der Bewegung übereinstimmen.

Eine Darstellung dieser Datensätze in einem geeigneten Visualisierungsverfahren als Sequenz ermöglicht dem Arzt eine genau Abschätzung der Bewegungsverläufe.

Da der vorher aufgenommene Referenzbilddatensatz mehrfach verwendet werden kann, ist so ein wiederholtes Studium von Bewegungsabläufen möglich, ohne dass eine zu hohe Dosis an ionisierender Röntgenstrahlung auf den Patienten einwirkt.

Verschiedene Varianten der Erfindung verwenden eindimensionale, zweidimensionale oder dreidimensionale Ultraschallbildgebung.

Eindimensionale Ultraschallbildgebung erzeugt sogenannte A-Scans, das sind eindimensionale Bilddaten entlang eines Ultraschallstrahls.

Zu jedem A-Scan wird dabei die Position des Ultraschallsensors vom Positionsbestimmungssystem übertragen. Durch die vorangegangene Kalibrierung ist damit die Richtung und Skalierung des Ultraschallsignals bekannt.

Bei zweidimensionaler Ultraschallbildgebung werden eine Reihe von A-Scans gemeinsam aufgenommen, die zu einem sogenannten B-Bilder zusammengefügt werden.

Je nach Ausführung des Positionsbestimmungssystem und Aufnahmefrequenz der B-Bilder kann dabei jeweils eine Position pro aufgenommenem B-Bild oder mehrere Positionen pro B-Bild bestimmt werden, so dass zu jedem A-Scan eine eigene Position berechnet werden kann. Letzteres ermöglich auch schnelle Bewegungen des Ultraschallsensors zu erfassen.

Bei dreidimensionaler Ultraschallbildgebung liefert das Ultraschallgerät einen dreidimensionalen Datensatz. Da auch diese Daten nacheinander per A-Scans aufgenommen werden, kann auch hier je nach Abtastrate des Positionsbestimmungssystems eine oder mehrere Positionen während der Aufnahme eines dreidimensionalen Ultraschalldatensatzes übertragen und zur Bestimmung der Koordinaten herangezogen werden.

## Patentansprüche

1. Verfahren zur Bestimmung der Lage eines Objektes oder eines Objektbereichs unter Verwendung eines mit einem ersten Bilderzeugungsverfahren erstellten ortsaufgelösten Referenzbilddatensatzes für ein Referenzbild und eines zweiten Bilderzeugungsverfahrens, insbesondere zur Bestimmung der Lage von Gewebeteilen und/oder Organen und/oder der Organstruktur bei einer navigierten Operation, mit den Schritten:
Identifizieren solcher im Referenzbild abgebildeter Bildbereiche, die bei dem zweiten Bilderzeugungsverfahren ein charakteristisches Abbildungsverhalten aufweisen,
Verwenden des zweiten Verfahrens zur Bilderzeugung zum Erzeugen eines weiteren ortsaufgelösten Bilddatensatzes für ein weiteres Bild des Objektes;
Ermitteln der Transformation zwischen dem weiteren Bilddatensatz und dem Referenzbilddatensatz, welche die identifizierten Bildbereiche in bestmögliche Übereinstimmung bringt;
Bestimmen der Lage des Objekts oder des Objektbereichs in der dem weiteren Bild entsprechenden Situation.

2. Verfahren nach Anspruch 1, bei welchem das Referenzbild eine dreidimensionale Ortsauflösung aufweist und das weitere Bild wenigstens eine zweidimensionale Ortsauflösung aufweist.

3. Verfahren nach einem der vorangegangenen Ansprüche, bei welchem das erste Bilderzeugungsverfahren ein Computertomographieverfahren, Röntgentomographieverfahren oder Magnetresonanztomographieverfahren ist und das zweite Bilderzeugungsverfahren ein Ultraschallverfahren ist.

4. Verfahren nach Anspruch 3, bei welchem das Ultraschallverfahren Messsignale mit eindimensionaler Ortsauflösung erzeugt, wobei das weitere Bild durch Überlagerung einer Mehrzahl der eindimensional ortsaufgelösten Messsignale erzeugt wird.

5. Verfahren nach Anspruch 3, bei welchem das Ultraschallverfahren Messsignale mit zweidimensionaler oder dreidimensionaler Ortsauflösung erzeugt.

6. Verfahren nach einem der vorangegangenen Ansprüche, wobei zum Erzeugen des weiteren ortsaufgelösten Bildes die mit dem zweiten Bilderzeugungsverfahren erzeugten Messsignale derart bearbeitet werden, dass die Bereiche mit charakteristischem Abbildungsverhalten hervorgehoben werden.

7. Verfahren nach Anspruch 6, bei dem der Bearbeitungsschritt das Durchführen einer punktweisen Grauwerttransformation umfasst.

8. Verfahren nach Anspruch 7, bei dem die punktweise Grauwerttransformation auf Grundlage einer quadratischen Kennlinie durchgeführt wird.

9. Verfahren nach einem der Ansprüche 6 bis 8, bei dem der Bearbeitungsschritt das Anwenden eines räumlichen Filters umfasst.

10. Verfahren nach einem der Ansprüche 2 bis 9, wobei die Ermittlung der Transformation durch Optimierung eines Gütekriteriums erfolgt, das auf dem identifizierten Bildbereich berechnet wird und dieser Bereich so identifiziert wurde, dass er die Oberfläche im dreidimensionalen Referenzbild darstellt, wobei der die identifizierten Oberflächen darstellende Datensatz aus dem dreidimensionalen Referenzbilddatensatz berechnet wird.

11. Verfahren nach Anspruch 10, bei dem der die Oberflächen darstellende Datensatz durch ein Segmentierungsverfahren aus dem dreidimensionalen Referenzbilddatensatz berechnet wird.

12. Verfahren nach Anspruch 11, bei dem als Segmentierungsverfahren ein Schwellwertverfahren verwendet wird, dessen Schwellwert so gewählt wird, dass die durch den Datensatz dargestellte Oberfläche eine Knochenoberfläche approximiert.

13. Verfahren nach einem der Ansprüche 10 bis 12, bei dem das Identifizieren von Bildbereichen in dem Referenzbild nur auf solchen Oberflächenbereichen durchgeführt wird, die einem zur Durchführung des zweiten Verfahrens verwendeten Ultraschallkopf zugewandt sind.

14. Verfahren nach einem der Ansprüche 10 bis 13, bei dem die Ermittlung der Transformation zwischen den Ultraschallbilddaten und den Referenzbilddaten unter Verwendung von Gewichtsfaktoren durchgeführt wird, die aus Normalenvektoren an Punkten der Oberfläche und den entsprechenden Vektoren der Ausbreitungsrichtung des Ultraschalls, vorzugsweise unter Bildung von Skalarprodukten, berechnet werden.

15. Verfahren nach Anspruch 14, bei dem die Gewichtsfaktoren durch Bildung der Skalarprodukte berechnet werden und proportional zu den Skalarprodukten sind.

16. Verfahren nach einem der Ansprüche 10 bis 15, bei dem das Gütekriterium für die Ermittlung der Transformation zwischen den Ultraschallbilddaten und den Referenzbilddaten die Summe der Beiträge aller Punkte der Oberfläche ist.

17. Verfahren nach einem der Ansprüche 3 bis 16, bei dem der Schritt des Identifizieren von Bildbereichen im Referenzbild das Identifizieren von Organen umfasst, welche im Ultraschallbild eine Abschattung erzeugen.

18. Verfahren nach Anspruch 17, bei dem die bei dem die Ermittlung der Transformation zwischen den Ultraschallbilddaten und den Referenzbilddaten so durchgeführt wird, dass die Summe der Grauwerte im Ultraschallbild innerhalb der Bildbereiche minimiert wird.

19. Verfahren nach einem der vorangegangenen Ansprüche zur Verwendung in einem System zur navigierten Operation unter Verwendung wenigstens eines Ultraschallsensors, um aus einem präoperativen Referenzbild und wenigstens einem aktuellen Ultraschallbild die aktuelle Lage von Gewebeteilen oder Organen zu ermitteln.

20. Verfahren nach einem der vorangegangenen Ansprüche, mit den weiteren Schritten:
Berechnen eines aktuellen Bilddatensatzes mit einem Informationsgehalt, der vergleichbar mit dem des Referenzbilddatensatzes ist, unter Verwendung des Referenzbilddatensatzes und der ermittelten aktuellen Transformation, und
Darstellen des dem aktuellen Bilddatensatz entsprechenden aktuellen Bildes.

21. Verfahren nach Anspruch 20, wobei im aktuellen Bild medizinische Instrumente gemeinsam mit den Gewebeteilen und/oder den Organen und/oder der Organstruktur abgebildet werden.

22. Verfahren nach Anspruch 20 oder 21, bei dem durch das zweite Bildverarbeitungsverfahren sukzessive weitere ortsaufgelöste Bilder während einer Bewegung des Patienten aufgenommen werden, wobei die sukzessiven aktuellen Bilder zur Darstellung der Bewegungsphase des Patienten verwendet werden.

23. Verfahren nach einem der Ansprüche 20 bis 22, wobei die Darstellung in Echtzeit erfolgt.
